# EUROPEAN PATENT APPLICATION

(11) **EP 1 205 552 A1**
(43) Date of publication of application: **15.05.2002**
(21) Application number: 00203947.7
(22) Date of filing: 09.11.2000
(51) Int. Cl.: C12N 15/31, C07K 14/315, C07K 16/12, A61K 38/16

(54) **Virulence of streptococci, involving ORFs from Streptococcus suis**

(71) Applicant: ID-Lelystad, Instituut voor Dierhouderij en Diergezondheid B.V., 8219 PH Lelystad (NL)
(72) Inventor: Smith, Hilda ELizabeth, 8241 AG Lelystad (NL)
(74) Representative: Prins, Adrianus Willem

(57) **Abstract**

The invention relates to the field of diagnosis of and vaccination against Streptococcal infections and to the detection of virulence markers of Streptococci. The invention provides a method for modulating virulence of a *Streptococcus* comprising modifying a genomic fragment of said *Streptococcus* wherein said genomic fragment comprises at least a functional part of a fragment identifiable by hybridisation in *Streptococcus suis* to a nucleic acid or fragment thereof as shown in figure 5.

SEQUENCE LISTING

<110> ID-Lelystad, Instituut voor Dierhouderij en Diergezondheid B.V.

<120> Virulence of Streptococci

<130> P53842EP00

<140> 00203947.7
<141> 2000-11-09

<160> 15

<170> PatentIn Ver. 2.1

<210> 1
<211> 30
<212> DNA
<213> Artificial Sequence

<220>
<223> Description of Artificial Sequence: primer

<220>
<221> misc_feature
<222> (1)..(30)

<400> 1
cgagctcgga agaattggtt attgcgcgtg                                   30

<210> 2
<211> 29
<212> DNA
<213> Artificial Sequence

<220>
<223> Description of Artificial Sequence: primer

<220>
<221> misc_feature
<222> (1)..(29)

<400> 2
cgggatcccg ggggatgacc tgttgcttg                                    29

<210> 3
<211> 31
<212> DNA
<213> Artificial Sequence

<220>

<223> Description of Artificial Sequence: primer

<220>
<221> misc_feature
<222> (1)..(31)

<400> 3
tcccccgggg gacaagcaac gggtcatccc c                                    31



<210> 4
<211> 30
<212> DNA
<213> Artificial Sequence

<220>
<223> Description of Artificial Sequence: primer

<220>
<221> misc_feature
<222> (1)..(30)

<400> 4
cgggatcccg gttgaatgcc cggcaaagcg                                      30



<210> 5
<211> 33
<212> DNA
<213> Artificial Sequence

<220>
<223> Description of Artificial Sequence: primer

<220>
<221> misc_feature
<222> (1)..(33)

<400> 5
tcccccgggg gagtcgtgtg tattcgacag cgg                                  33



<210> 6
<211> 6
<212> DNA
<213> Artificial Sequence

<220>
<223> Description of Artificial Sequence: putative
      promoter sequence

<220>
<221> misc_feature

<222> (1)..(6)

<400> 6
tggaca                                                                 6



<210> 7
<211> 6
<212> DNA
<213> Artificial Sequence

<220>
<223> Description of Artificial Sequence: putative
      promoter sequence

<220>
<221> misc_feature
<222> (1)..(6)

<400> 7
tacaat                                                                 6



<210> 8
<211> 7
<212> DNA
<213> Artificial Sequence

<220>
<223> Description of Artificial Sequence: putative
      promoter sequence

<220>
<221> misc_feature
<222> (1)..(7)

<400> 8
atggaca                                                                7



<210> 9
<211> 6
<212> DNA
<213> Artificial Sequence

<220>
<223> Description of Artificial Sequence: putative
      promoter sequence

<220>
<221> misc_feature
<222> (1)..(6)

<400> 9
tggtca                                                                  6

<210> 10
<211> 233
<212> PRT
<213> Streptococcus suis

<220>
<221> SITE
<222> (1)..(233)
<223> /note="ORF2 V10"

<400> 10
Met Leu Pro His Asn Glu Ala Asp Leu Cys Leu His Ile Met Ser Pro
1               5                   10                  15

Arg Val Gly Thr Leu Val Leu Ala Glu Ser Ser Ala Val Asn His Cys
            20                  25                  30

Ile Arg Cys Arg Ile His Thr Thr Ala Pro Phe Phe Glu Lys Gly Ala
        35                  40                  45

Phe Phe Met Glu Lys Lys Ile Pro Lys Leu Thr Val Gln Leu Leu Ala
    50                  55                  60

Ala Ile Ala Met Thr Leu Ala Leu Val Met Ile Val Glu Asn Tyr Phe
65                  70                  75                  80

Ser Ile Arg Ile Ser Asp Thr Leu Gln Val Gln Phe Thr Phe Ile Pro
                85                  90                  95

Asn Thr Ile Leu Gly Ala Ile Ala Gly Pro Val Trp Ala Ala Val Phe
            100                 105                 110

Ala Ala Ile Ser Asp Pro Val Phe Val Leu Phe Ser Gly Gln Thr Val
        115                 120                 125

Leu Phe Thr Trp Ile Leu Ile Glu Ala Val Ser Ala Phe Ile Tyr Gly
    130                 135                 140

Trp Phe Phe Tyr Arg Lys Pro Leu Asp Thr Lys Asn Lys Ala Asp Trp
145                 150                 155                 160

Leu Tyr Val Ala Gly Val Val Val Leu Ile Gln Val Val Ile Ser Phe
                165                 170                 175

Ile Met Thr Pro Ile Ala Leu His Phe His Phe Gly Thr Pro Trp Ile
            180                 185                 190

Val Leu Tyr Ser Ser Arg Leu Ile Lys Ala Val Phe Glu Ile Pro Leu
        195                 200                 205

Arg Ile Val Val Thr Met Leu Val Leu Pro Ser Leu Gln Lys Ile Pro

210                 215                 220

Glu Leu Ala Lys Leu Met Gly Ile Lys
225                 230

<210> 11
<211> 183
<212> PRT
<213> Streptococcus suis

<220>
<221> SITE
<222> (1)..(183)
<223> /note="ORF2 V735"

<400> 11
Met Glu Lys Lys Ile Pro Lys Leu Thr Val Gln Leu Leu Ala Ala Ile
1               5                   10                  15

Ala Met Thr Leu Ala Leu Val Met Ile Ala Glu Asn His Phe Ser Val
            20                  25                  30

Arg Leu Ser Asp Thr Leu Gln Val Gln Phe Thr Phe Ile Pro Asn Thr
        35                  40                  45

Ile Leu Gly Ala Ile Ala Gly Pro Val Trp Ala Ala Val Phe Ala Ala
    50                  55                  60

Ile Ser Asp Pro Ala Phe Val Leu Phe Ser Gly Gln Ser Met Leu Phe
65                  70                  75                  80

Ser Phe Ile Leu Ile Glu Ala Val Ser Ala Phe Ile Tyr Gly Trp Phe
                85                  90                  95

Phe Tyr Arg Lys Pro Leu Asp Thr Lys Asn Lys Ala Asp Trp Leu Tyr
            100                 105                 110

Val Ala Gly Val Val Val Leu Ile Gln Val Val Ile Ser Phe Ile Met
        115                 120                 125

Thr Pro Ile Ala Leu His Phe His Phe Gly Thr Pro Trp Ile Val Leu
    130                 135                 140

Tyr Ser Ser Arg Leu Ile Lys Ala Val Phe Glu Ile Pro Leu Arg Ile
145                 150                 155                 160

Val Val Thr Met Leu Val Leu Pro Ser Leu Gln Lys Ile Pro Glu Leu
                165                 170                 175

Ala Lys Leu Met Gly Ile Lys
            180

<210> 12
<211> 418
<212> PRT
<213> Streptococcus suis

<220>
<221> SITE
<222> (1)..(418)
<223> /note="ORF3 V10"

<400> 12
Met Asn Tyr Gln Glu Thr Arg Arg Trp Leu Ser Ser Arg Pro Ala Ser
1               5                   10                  15

Asp Leu Glu Asn Gly Val Ala Arg Val Asn Trp Ile Leu Glu Arg Leu
            20                  25                  30

Asp Asn Pro Gln Leu Gln Val Pro Thr Val His Phe Val Gly Thr Asn
        35                  40                  45

Gly Lys Gly Ser Thr Leu Asn Ala Leu Gln Ser Ile Leu Gln Ser Ser
    50                  55                  60

Asp Tyr Thr Val Gly Arg Phe Thr Ser Pro Ser Ile Ile Asp Phe Arg
65                  70                  75                  80

Glu Gln Ile Val Tyr Gln Gln Glu Met Ile Ser Glu Glu Asp Phe Ala
                85                  90                  95

Arg Ile Val Thr Asp Leu Gln Pro Leu Ile Glu Asp Leu Asp Gln Thr
            100                 105                 110

Ala Gly Leu Asp Ala Ile Ser Glu Phe Glu Ile Val Val Val Ala Met
        115                 120                 125

Phe Val Tyr Phe Ala His Tyr Gln Arg Pro Asp Ile Leu Leu Val Glu
    130                 135                 140

Ala Gly Met Gly Gly Leu Gln Asp Ala Thr Asn Val Leu Ala Pro Leu
145                 150                 155                 160

Ala Val Val Cys Pro Ser Ile Gly Leu Asp His Gln Ala Phe Leu Gly
                165                 170                 175

Glu Thr His Ala Ala Ile Ala Arg His Lys Val Ala Val Leu Arg Glu
            180                 185                 190

Gly Val Pro Leu Ile Tyr Ala Thr Asp Gln Pro Glu Val Glu Thr Val
        195                 200                 205

Phe Glu Glu His Ala Cys Gln Leu Gln Ser Pro Thr Tyr Ala Val Gly
    210                 215                 220

Arg Glu Ile Leu Leu Glu Asn Ser Arg Ala Gly Phe Ala Val Ser Ser
225                 230                 235                 240

Pro Leu Gly Arg Val Glu Glu Leu Thr Leu Gln Met Gln Gly Arg His
                245                 250                 255

Gln Glu Val Asn Ala Ala Leu Ala Val Thr Thr Ala Gln Leu Ile Lys
            260                 265                 270

Pro His Phe Pro Thr Ile Thr Asn Glu Thr Ile Arg Gln Gly Leu Ser
        275                 280                 285

Gln Ala Ile Trp Pro Gly Arg Leu Glu Leu Ile Arg Pro Asn Leu Met
    290                 295                 300

Ile Asp Gly Ala His Asn Asn Glu Ser Ile Ala Val Leu Thr Gln Leu
305                 310                 315                 320

Leu Glu Glu Lys Tyr Ala Asp Arg Asp Ile Glu Ile Leu Phe Ala Ala
                325                 330                 335

Ile Asn Thr Lys Pro Val Asp Gln Met Leu Ser Gln Leu Ser Gln Phe
            340                 345                 350

Gly Pro Val Ser Val Thr Thr Phe Asp Asp Phe Arg Ala Val Gln Leu
        355                 360                 365

Glu Asp Tyr Pro Ser Gly Tyr Glu Arg Val Gln Thr Tyr Gln Glu Trp
    370                 375                 380

Val Glu Gln Ala Asp Leu Asp Asn Pro Lys Lys Leu Tyr Leu Ile Thr
385                 390                 395                 400

Gly Ser Leu Tyr Phe Ile Thr Tyr Val Arg Lys Tyr Ile Leu Glu Glu
                405                 410                 415

Leu Val

<210> 13
<211> 418
<212> PRT
<213> Streptococcus suis

<220>
<221> SITE
<222> (1)..(418)
<223> note="ORF3 V735"

<400> 13
   Met Asn Tyr Gln Glu Thr Arg Arg Trp Leu Ser Ser Arg Pro Ala Ser
     1               5                  10                  15
   Asp Leu Glu Asn Gly Val Ala Arg Val Asn Trp Ile Leu Glu Arg Leu
                20                  25                  30
   Asp Asn Pro Gln Leu Gln Val Pro Thr Val His Phe Val Gly Thr Asn
   35 40 45
   Gly Lys Gly Ser Thr Leu Asn Ala Leu Gln Ser Ile Leu Arg Ser Ser
   50 55 60
   Asp Tyr Thr Val Gly Arg Phe Thr Ser Pro Ser Ile Ile Asp Phe Arg
   65 70 75 80
   Glu Gln Ile Val Phe Glu Gln Glu Met Ile Ser Glu Glu Asp Phe Ala
   85 90 95
   Arg Ile Val Thr Asp Leu Gln Pro Leu Ile Glu Asp Leu Asp Gln Thr
   100 105 110
   Ala Gly Leu Asp Ala Ile Ser Glu Phe Glu Ile Val Val Val Ala Met
   115 120 125
   Phe Val Tyr Phe Ala His Tyr Gln Arg Pro Asp Ile Leu Leu Val Glu
   130 135 140
   Ala Gly Met Gly Gly Leu Gln Asp Ala Thr Asn Val Leu Ala Pro Leu
   145 150 155 160
   Ala Val Val Cys Pro Ser Ile Gly Leu Asp His Gln Ala Phe Leu Gly
   165 170 175
   Glu Thr His Ala Ala Ile Ala Arg His Lys Val Ala Val Leu Arg Glu
   180 185 190
   Arg Val Pro Leu Leu Tyr Ala Thr Asp Gln Ser Glu Val Val Ala Ala
   195 200 205
   Phe Glu Asp His Ala Ser Gln Leu Gln Ser Pro Thr Tyr Ala Val Gly
   210 215 220
   Arg Glu Ile Leu Leu Glu Asn Ser Arg Ala Gly Phe Ala Val Ser Ser
   225 230 235 240
   Thr Leu Gly Arg Val Glu Glu Leu Thr Leu Gln Met Gln Gly Arg His
   245 250 255
   Gln Glu Val Asn Ala Ala Leu Ala Val Thr Thr Ala Gln Leu Leu Ser
   260 265 270
   Pro Asp Phe Pro Thr Ile Thr Asn Glu Thr Ile Arg Gln Gly Leu Ser
   275 280 285
   Gln Ala Ile Trp Pro Gly Arg Leu Glu Leu Ile Arg Pro Asn Leu Met
   290 295 300
   Ile Asp Gly Ala His Asn Asn Glu Ser Ile Ala Val Leu Thr Gln Leu
   305 310 315 320
   Leu Glu Glu Lys Tyr Ala Asp Arg Asp Ile Glu Ile Leu Phe Ala Ala
   325 330 335
   Ile Asn Thr Lys Pro Val Asp Gln Met Leu Ser Gln Leu Ser Gln Phe
   340 345 350
   Gly Pro Val Ser Val Thr Thr Phe Asp Asp Phe Arg Ala Val Gln Leu
   355 360 365
   Gly Asp Tyr Pro Ser Gly Tyr Glu Arg Val Gln Thr Tyr Gln Glu Trp
   370 375 380
   Leu Glu Gln Val Asp Leu Asp Asn Pro Lys Gln Leu Tyr Leu Ile Thr
   385 390 395 400
   Gly Ser Leu Tyr Phe Ile Thr Tyr Val Arg Lys Tyr Ile Leu Glu Glu
   405 410 415
   Leu Val

<210> 14
<211> 2973
<212> DNA
<213> Streptococcus suis

<220>
<221> misc_feature
<222> (1)..(2973)
<223> /note="V10 sequence fragment"

<400> 14
ggatcctgct atcattcctt atttgattgc gaatgttgaa gaactgaaag atgctgcaga 60

cgttgttaac atgttgaata aacagtcagg cttattcggt gtatctggct tctcaagtga 120

tatgcgtgat attgaagcag gtatccaagc tcacaatcca gatgcagtgt tggcctacaa 180

tattttcatt gaccgtatta agaaatttat cggtcagtat cttgcagttt taaatggggc 240

agatgctatt gtcttcaccg ctggtatggg tgaaaatgca ccgcttatgc gcaatgacgt 300

agtagaaggt ttgtcttggt ttggtattga gttggaccta caaaaaaatg tattcggcaa 360

ctatggtgac atttcaacgg cagaatcaaa agttcgtgtc ttggttattc cgacggatga 420

agaattggtt attgcgcgtg aagtggaacg cttgaaataa gaaaaactaa ctggtagtcg 480

gagactgcca gtttctctta tagtttatac ctttagaaag gtatagtttt tagcaagtgg 540

acaaaatata tagtgtgtga tacaatagac tagcaaagaa atttgcacag agtagatggt 600

ttgcgtcaag tgtatgtgga tgggatgttg ccacataacg aagctgatct ttgcttgcat 660

ctgatgtctc ctagagtagg aacattggtc ctggctgaga gtagcgcggt aaaccattgc 720

atccgctgtc gaatacacac gacagctcca ttttttgaaa aaggagcatt ttttatggaa 780

aagaaaattc caaaactaac ggtgcagttg ttggctgcta ttgcgatgac ccttgccttg 840

gtcatgattg tagagaacta tttctctatt cggatttctg atactttaca ggttcagttt 900

accttcattc ccaatactat tttgggagct attgcgggtc cagtttgggc agctgtcttt 960

gcggctattt cagacccagt ctttgtcttg tttagcgggc aaacggtcct cttcacttgg 102
0
attttgattg aggcggtatc ggcatttatc tacggctggt tcttctatcg aaaaccgcta 108
0
gacaccaaga acaaggctga ttggctctat gtggctggtg tagttgtctt gattcaggtt 114
0
gtgatttcct ttatcatgac accgattgcc ctccatttcc attttggaac accttggatt 120
0
gttctgtata gcagtcgctt gattaaggca gtttttgaaa ttccattacg cattgtcgtg 126
0
accatgcttg tcttgccaag tttacaaaaa atacctgaat tggccaagtt aatgggcatt 132
0
aaataaaaca gtatcaagca acaggtcatc cccctgttgc tacttttgta gagagggaat 138
0
catgaattat caagaaactc gccggtggct atctagtcgt cctgcatcag atttagaaaa 144
0
tggcgttgca cgtgtcaact ggattttaga acgcttggac aatccccagc ttcaagtgcc 150
0
gaccgtacac tttgtgggca caaatggcaa gggctcgacc ctcaacgcct tacagtctat 156
0
cttgcagtct tcggattaca ccgtcggccg ctttacatca ccgtctatca ttgattttcg 162
0
agagcagatt gtctaccagc aggagatgat ttcggaggaa gattttgcga ggattgtgac 168
0
agacttgcaa cccttgatcg aggacttgga ccagacggct ggactggatg ccatctcgga 174
0
gtttgagatt gtagtagtgg ctatgtttgt ctactttgcc cactaccagc gtcccgatat 180
0
tctcttggtg gaggccggca tgggtggttt gcaggatgcg accaatgtcc ttgccccctt 186
0
ggcagtagtt tgtccgtcca tcggtttgga ccatcaggca tttttgggag agacccacgc 192
0
tgctatagcc cgtcacaagg tcgccgtctt gcgtgagggg gttccgctca tctatgcgac 198
0
cgaccagcca gaagtggaga cagtatttga ggagcatgcc tgtcagcttc agagtccgac 204
0
ctatgcggtg ggcgggagaa ttcttttgga aaatagcaga gcaggctttg cagtttcaag 210
0
tcctctcggc cgtgtggaag agttaacact acagatgcag ggtcgtcacc aggaggtcaa 216
0
tgcagccttg gcagtgacaa cagctcagct cattaaacct cattttccaa caattaccaa 222
0
tgaaaccatc cgccagggct tgtcccaagc catctggccg ggtcgcttag agttgattag 228
0
gcctaatctc atgattgacg gtgcccacaa taatgaaagt atcgccgtcc tgacacaact 234
0
cttggaagaa aagtatgctg acaggatatt tgaaatcctc tttgcggcca tcaataccaa 240
0
gccagtggac cagatgttgt cccagcttag ccaatttgga cctgttagcg tgacgacctt 246

0
tgacgatttc agagcggtac agttagaaga ttatccgtca ggctatgaac gagttcagac 252
0
ctatcaggag tgggtggagc aggcggactt ggacaatccc aaaaaactct acctgattac 258
0
aggctcgcta tatttcatta cctatgtgag gaagtacatt ttagaagaac ttgtttagaa 264
0
aaaaaaggct ttgccgggca ttcaacccag caaagtcttt tgttttaata atttttaatc 270
0
aaatcaaccg ttgagcggtc tagtttttta acgatggtct gcaagaaggc ttgggcctct 276
0
aagaagtcat ccatgctgta gagagtttga tgtgaatgga tgtagcgagc gcagacaccg 282
0
atagttgttg atggaacacc atgatttttc aagtgggctg caccagcatc tgttccacct 288
0
ttaccacagt agtattggaa tttgacacct gcttcttcgg cagttgtgag gaggaagtct 294
0
ttcatgtttt ttaacatgat gtgacctgga tcc 297
3

<210> 15
<211> 3098
<212> DNA
<213> Streptococcus suis

<220>
<221> misc_feature
<222> (1)..(3098)
<223> /note="V735 sequence fragment"

<400> 15
ctgcagatgt tgtgaacatg ttgaataaac agtcaggctt gttcggtgta tctggcttct 60

caagtgatat gcgtgatatt gaagcaggca tccaagctca caatccagat gcagtgttgg 120

cctacaatat tttcattgac cgtattaaga aatttatcgg tcagtatctt gcagttttaa 180

atggggcaga tgctattgtc ttcacggctg gtatgggtga aaatgcaccg cttatgcgca 240

atgacgtagt agaaggcttg tcttggtttg gtattgagtt ggacccacaa aaaaatgtat 300

ttggcaacta tggtgacatt tcaacggcag aatcaagggt tcgtgtcttg gttattccga 360

cggatgaaga attggttatt gcgcgtgaag ttgaacgttt gaaataagaa aaactaactg 420

gtagtcggag actgccggtt tctcttatag tttatacctt tagaaaggta tagtttttag 480

caagtggtca aaatatatag tgtgtgatac aatagactag caaagaaatt tgcacagagt 540

agatggtttg cgtcaagtgt atgtggatgg gatgttgcca cataacgaag ctgatctttg 600

cttgcatctg atgtctccta gagtaggaac attggatctg gctgagagta gcgcggtaaa 660

ccattgcatc cgctgtcgaa tacacacgac agctccattt tttgaaaagg agcatttttt 720

atggaaaaga agattccaaa actaacggtg cagttgttgg ctgctattgc gatgactctt 780

gccttggtca tgattgcgga gaaccatttt tctgttcgtc tttctgatac cttgcaggtc 840

cagtttacct ttatccctaa tactatttta ggtgcgattg ctggtcctgt ttgggctgct 900

gtatttgcgg cgatttcaga cccagctttt gtcttgttta gtggacagag catgcttttt 960

agttttatct tgattgaggc ggtatcggct tttatctatg gctggttctt ctatcgaaaa 102
0
ccgctagaca ccaagaacaa ggctgattgg ctctatgttg caggggttgt tgtcttgatt 108
0
caggttgtga tttcctttat catgacaccg attgccctcc atttccattt tggaacacct 114
0
tggattgttc tgtatagcag tcgcttgatt aaggcggttt ttgaaattcc attacgcatt 120
0
gtcgtgacta tgcttgtctt accaagttta caaaaaatac ctgaattggc taagttaatg 126
0
ggcattaaat aaaacagtat caagcaacag gtcatccccc tgttgctgct tttgtagaga 132
0
gggaatcatg aattatcaag aaactcgccg gtggctatct agtcgtcctg catcagattt 138
0
agaaaatggc gttgcacgtg tcaactggat tttggaacgc ttggacaatc cccagcttca 144
0
agtgccgacc gttcacttcg taggtacaaa tggcaagggc tcgaccctca acgccttaca 150
0
gtctatctta cggtcttcgg attacaccgt cggtcgcttt acctcaccgt ctatcattga 156
0
ttttcgagag cagattgtat ttgagcagga gatgatttcg gaggaagatt ttgcaaggat 162
0
tgtgacagac ttgcaaccct tgattgagga cttggaccag acggctggac tggatgccat 168
0
ctcggagttt gagattgtag tagtggctat gtttgtctac tttgcccact accagcgtcc 174
0
cgacattctc ttggtggagg cgggcatggg tggtttgcag gatgcgacca atgtccttgc 180
0
cccattggca gtagtttgcc cgtccatcgg cttggaccat caggcttttt tgggagagac 186
0
ccacgctgct atagcccgtc acaaggttgc tgtcttgcgt gagcgggttc ccctcctcta 192
0
tgcgaccgac cagtcagaag tggtggcagc atttgaggat cacgccagtc agcttcagag 198
0
tccgacctat gcggtgggac gggagattct tttggaaaat agcagagcag gctttgctgt 204
0
ttcaagtact ctcggccgtg tggaagaatt aacactgcag atgcagggtc gtcaccagga 210
0
ggtcaatgca gccttggcag tgacaacagc tcagcttctc agccctgatt ttccaacaat 216
0
taccaatgaa accatccgcc agggcttgtc ccaagccatc tggccgggcc gcttagagtt 222
0
gattaggcct aatctcatga ttgacggtgc ccacaataat gaaagtatcg ccgtcctgac 228
0
acaactcttg gaagaaaagt atgctgacag ggatattgaa atcctctttg cggccatcaa 234
0
taccaagcca gtggaccaga tgttgtccca gcttagccaa tttggacctg ttagcgtgac 240
0

gacctttgac gatttcagag cggtacagtt aggagattat ccgtcaggct atgaacgagt 246
0
tcagacctat caggagtggt tggagcaggt ggacttggac aatcccaaac aactctacct 252
0
gattacaggc tcgctatatt tcattaccta tgtgaggaag tacattttag aagaacttgt 258
0
atagaaaaaa ggctttgccg ggcattcaac ccagcaaagt cttttgtttt aataattttt 264
0
aatcaaatca accgttgagc ggtctagttt tttaacgatg gtctgcaaga aggcttgggc 270
0
ctctaagaag tcatccatgc tgtagagagt ttgatgtgaa tggatgtagc gagcgcagac 276
0
accgatagtt gttgatggaa caccatggtt tttcaagtgg gctgcaccgg catctgttcc 282
0
acctttacca cagtagtatt ggaatttgac acctgcttct tcggcagttg tgaggaggaa 288
0
gtctttcatg ttttttagca tgatgtggcc tgggtcatag aaacgaagca gagttccgtc 294
0
accaattttt ccttggtcgc cataaatatc acctgcgggc gagcaatcaa cagcgaggaa 300
0
aatgtctgga ttgaacttgg ttgtagaggc atgagcacca cgaagaccaa cctcttactt 306
0
gcacattggc cccagcaatc aactgatttg caaagctt 309
8

## Description

The invention relates to the field of diagnosis of and vaccination against Streptococcal infections and to the detection of virulence markers of Streptococci.

*Streptococcus species,* of which there are a large variety causing infections in domestic animals and man, are often grouped according to Lancefield's groups. Typing according to Lancefield occurs on the basis of serological determinants or antigens that are among others present in the capsule of the bacterium and allows for only an approximate determination, often bacteria from a different group show cross-reactivity with each other, while other Streptococci can not be assigned a group-determinant at all. Within groups, further differentiation is often possible on the basis of serotyping; these serotypes further contribute to the large antigenic variability of Streptococci, a fact that creates an array of difficulties within diagnosis of and vaccination against Streptococcal infections.

Lancefield group A *Streptococcus* (GAS, *Streptococcus pyogenes*), are common with children, causing nasopharyngeal infections and complications thereof. Among animals, especially cattle are susceptible to GAS, whereby often mastitis is found.

Lancefield group B *Streptococcus* (GBS) are most often seen with cattle, causing mastitis, however, human infants are susceptible as well, often with fatal consequences. Group B streptococci (GBS) constitute a major cause of bacterial sepsis and meningitis among human neonates born in the United States and Western Europe and are emerging as significant neonatal pathogens in developing countries as well.

Lancefield group C infections, such as those with S. *equi, S. zooepidemicus, S. dysgalactiae*, and others are mainly seen with horse, cattle and pigs, but can also cross the species barrier to humans.

Lancefield group D (*S. bovis*) infections are found with all mammals and some birds, sometimes resulting in endocarditis or septicaemia.

Lancefield groups E, G, L, P, U and V *(S. porcinus, S, canis, S. dysgalactiae*) are found with various hosts, causing neonatal infections, nasopharyngeal infections or mastitis.

Within Lancefield groups R, S, and T, (and with ungrouped types) *S. suis* is found, an important cause of meningitis, septicemia, arthritis and sudden death in young pigs. Incidentally, it can also cause meningitis in man.

Ungrouped *Streptoccus species,* such as *S*. *mutans*, causing carries with humans, *S*, *uberis,* causing mastitis with cattle, and *S. pneumonia,* causing major infections in humans, and *Enterococcus faecilalis* and *E. faecium*, further contributed to the large group of Streptococci. *Streptococcus pneumoniae* (the pneumococcus) is a human pathogen causing invasive diseases, such as pneumonia, bacteraemia, and meningitis.

Little is known about the pathogenesis of the disease caused by Streptococci. Various cellular components, such as muramidase-released protein (MRP) extracellular factor (EF) and cell-membrane associated proteins, fimbriae, haemagglutinins, and haemolysin have been suggested as virulence factors. However, the precise role of these protein components in the pathogenesis of the disease remains unclear. It is however, well known and generally accepted that the polysaccharidic capsule of various Streptococci and other gram-positive bacteria plays an important role in pathogenesis. The capsule enables these micro-organisms to resist phagocytosis and is therefore regarded as an important virulence factor or marker.

In particular, *Streptococcus suis* is an important cause of meningitis, septicemia, arthritis and sudden death in young pigs (6, 31). It can also cause meningitis in man (2). Attempts to control the disease are still hampered by the lack of sufficient knowledge about the pathogenesis of the disease and the lack of effective vaccines and sensitive diagnostic methods.

So far, 35 serotypes of S. *suis* are described (8, 9, 10). Virulence of *S*. *suis* can differ within and among serotypes (29, 30, 32, 33). Worldwide *S. suis* serotype 2 is the most frequently isolated serotype. Within *S. suis* serotype 2, pathogenic, weak-pathogenic and non-pathogenic strains can be found (32, 33). The pathogenic strains cause severe clinical signs of disease in pigs and large numbers of bacteria can be reisolated from the central nervous system (CNS) and the joints after experimental infection (32, 33). The weak-pathogenic strains cause only mild clinical signs of disease and only infrequently bacteria can be reisolated from the CNS and the joints after experimental infection (32, 33). The non-pathogenic strains are completely avirulent in young pigs after experimental infection (32, 33).

The 136-kDa muramidase-related protein (MRP) and the 110-kDa extracellular factor (EF) are generally considered as important virulence markers for *S. suis* serotype 2 strains isolated in Europe and the United States (3, 7, 16, 21, 28, 35). However, differences in virulence between pathogenic, weak-pathogenic and non-pathogenic strains cannot exclusively be explained by differences in their MRP and EF expression patterns (26). In addition, it is known that the capsule of *Streptococcus suis* serotype 2 is an important virulence factor (23). However, since both pathogenic, weak-pathogenic and non-pathogenic strains seemed to be fully encapsulated after growth *in vitro* and *in vivo,* it is not likely that the level of encapsulation of these fully encapsulated strains is associated with their difference in virulence.

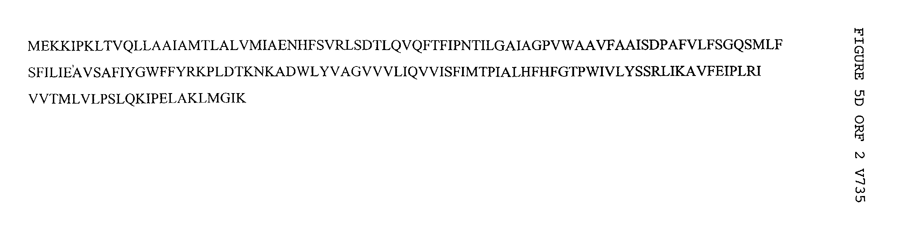

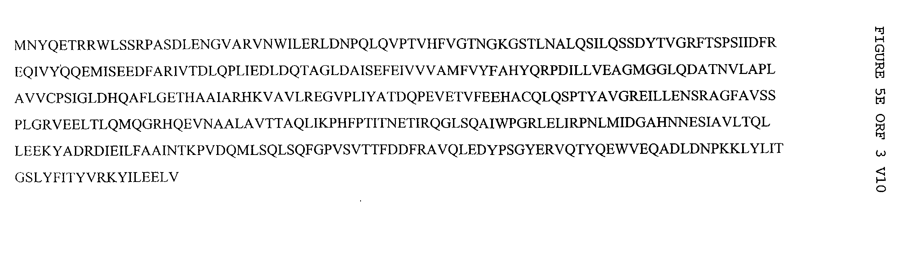

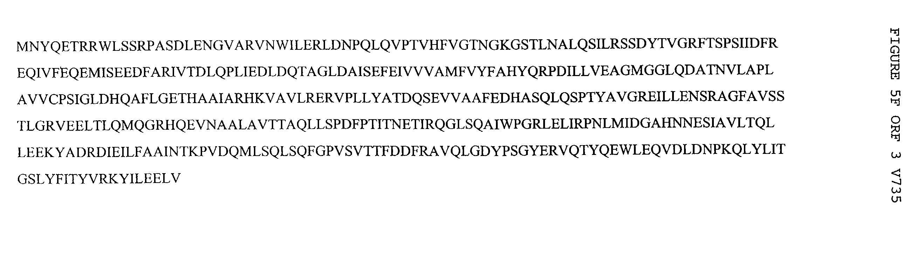

The invention provides a method for modulating virulence of a *Streptococcus* comprising modifying a genomic fragment of said *Streptococcus* wherein said genomic fragment comprises at least a functional part of a fragment identifiable by hybridisation in *Streptococcus suis* to a nucleic acid or fragment thereof as shown in figure 5. To get insight in the differences between pathogenic, weak-pathogenic and non-pathogenic strains, that determined their difference in virulence, the invention provides an *in vivo* complementation system whereby virulence can be modified by modifying said fragment. For example, within *S. suis* serotype 2 pathogenic, weak-pathogenic and non-pathogenic strains can be found. We introduced a genomic library of a pathogenic strain into a weak-pathogenic strain. After infection of the library into young piglets pathogenic transformants were selected. One specific transformant, containing a 3-kb fragment of the pathogenic strain, V10, appeared to be dominantly enriched in diseased pigs. The observed enrichment was not tissue specific. The selected fragment, when introduced into two different weak-pathogenic strains, increased the virulence of these strains considerably. In particular, the fragment herein identified as ORF 2, or functional fragments thereof is an important virulence factor. In contrast, introduction of the corresponding fragment of a weak-pathogenic strain had only minor effects on virulence. Thereby, the invention also provides a method for assaying virulence of a *Streptococcus* comprising assaying a genomic fragment of said *Streptococcus* wherein said genomic fragment comprises at least a functional part of a fragment identifiable by hybridisation in *Streptococcus suis* to a nucleic acid or fragment thereof as shown in figure 5, in particular the ORF 2 fragment. Nucleotide sequence analysis of the selected fragment of the pathogenic strain revealed the presence of two potential open reading frames both of which were found to be mutated in the corresponding fragment of the weak-pathogenic strain. It was previously shown by ribotyping and random amplified polymorphic DNA analysis (RAPD) assays, that pathogenic and weak-pathogenic strains of *S. suis* serotype 2 are genetically closely related, whereas non-pathogenic strains showed a high degree of genetic heterogeneity (5, 24). We constructed a genomic library of the pathogenic S. *suis* strain 10 in plasmids and introduced the plasmid library into the weak-pathogenic reference strain of *S*. *suis* serotype 2, strain S735 (34). Pigs were inoculated intravenously with the recombinants and bacteria were recovered from the CNS and the joints of diseased pigs. The re-isolated bacteria were subsequently analyzed for their plasmid content and their virulence. With this approach we identified a DNA fragment of a pathogenic serotype 2 strain that transformed weak-pathogenic strains into highly pathogenic strains. This fragment as provided herein comprises a genetic determinant important for virulence. Said fragment is in other Streptococci identifiable by for example hybridisation experiments such as Northern or Southern blotting or by amplification experiments such as PCR using primers and/or probes derivable from a nucleic acid as provided herein.

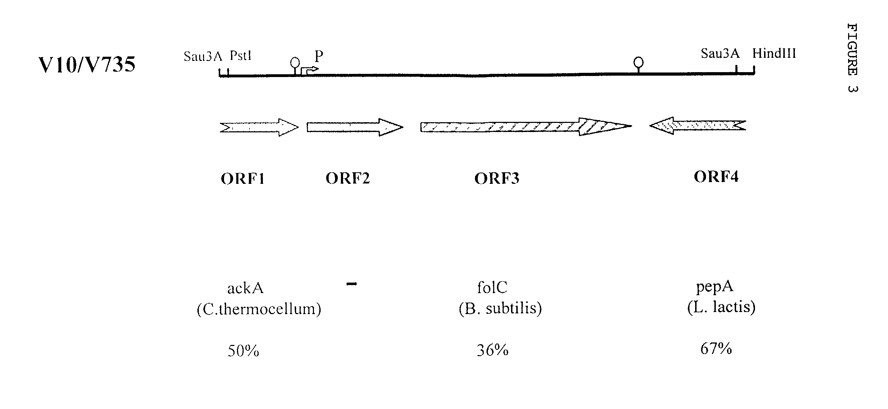

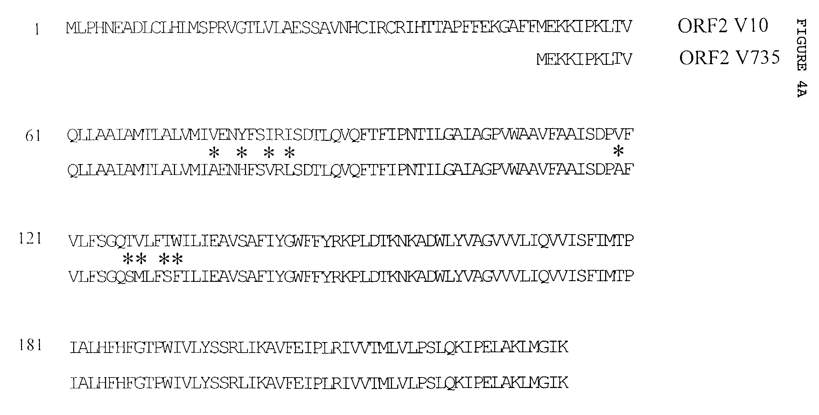

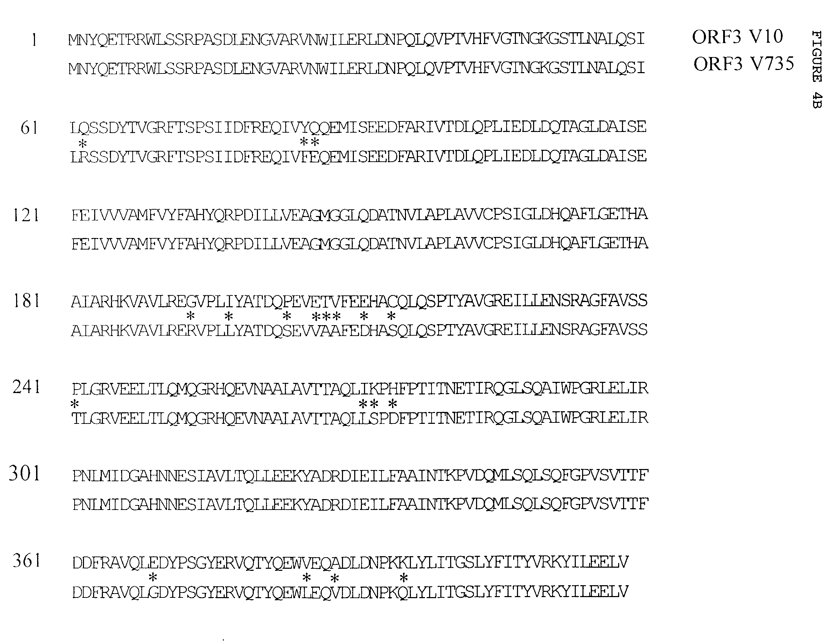

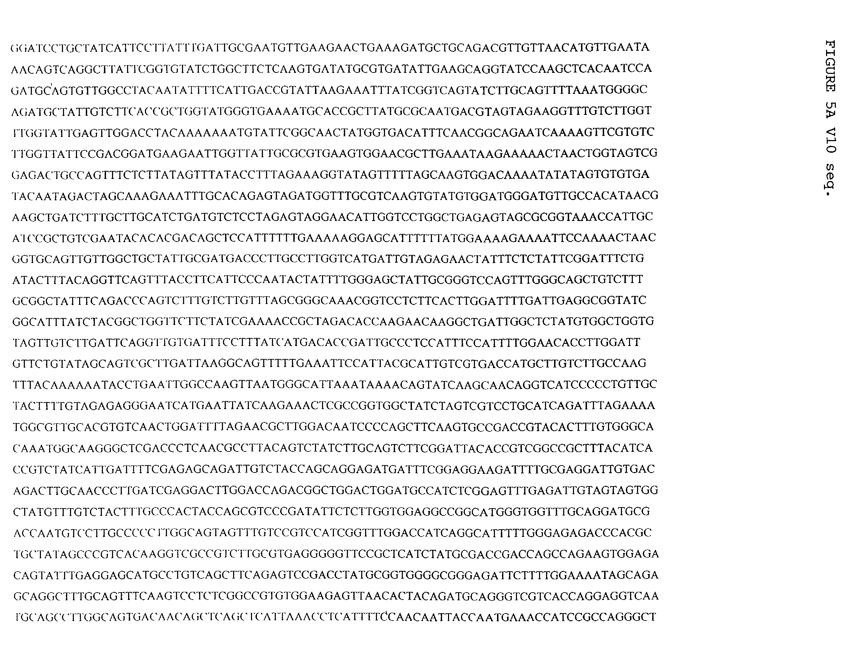

With the fragment, and parts thereof, such as the open reading frames identified in figure 3, a virulence marker is provided herein. Said marker is associated with an isolated and/or recombinant nucleic acid as provided herein and derivable from *Streptococcus* and identifiable by hybridisation in *Streptococcus* (preferably *S*. *suis)* to a nucleic acid or fragment thereof as shown in figure 5.

The invention also provides a vector comprising a nucleic acid according to the invention, and a host cell comprising a nucleic acid or a vector according to the invention. Such a host cell preferably comprises an easily modifiable organism such as E. coli, however, other host cells, such as recombinant *Streptococcus* (preferably derived from one of the grouped or ungrouped *Streptococci* as identified herein above) comprising a vector or nucleic acid according to the invention are herein also provided. In particular, recombinant *Streptococcus* as provided herein is useful for inclusion in a vaccine.

Furthermore, the invention provides a vaccine comprising a nucleic acid or a vector or a host cell according to the invention, and use of such a vaccine in the prevention and/or treatment of Streptococcal infections.

Also provided is a protein or fragment thereof encoded by a nucleic acid according to the invention, such as a protein encoded by ORF 2 or ORF 3 as disclosed herein or functional, i.e. antigenic fragment thereof. The invention also provides an antibody directed against a protein or fragment thereof according to the invention and an antigen reactive with such an antibody, for example comprising a protein or fragment. Such a protein or fragment thereof need not be obtained by recombinant means only, synthesis of peptides, according to their amino acid sequence, is equally well possible. Such antigens and antibodies as provided herein can be used in a diagnostic test comprising an antibody according to the invention, or within a vaccine or diagnostic test comprising an antigen according to the invention. Such vaccines and diagnostic tests can be used in the field of diagnosis of and vaccination against Streptococcal infections and for the detection of virulence markers of Streptococci.

### Detailed description

Bacterial strains and growth conditions. The bacterial strains and plasmids used in this study are listed in Table 1. S. *suis* strains were grown in Todd-Hewitt broth (code CM189, Oxoid), and plated on Columbia agar blood base (code CM331, Oxoid) containing 6% (v/v) horse blood. If required, antibiotics were added at the following concentrations: erythromycin, 1 µg/ml. *E.coli* strains were grown in Luria broth (18) and plated on Luria broth containing 1.5% (w/v) agar. If required, 200 µg/ml of erythromycin was added.

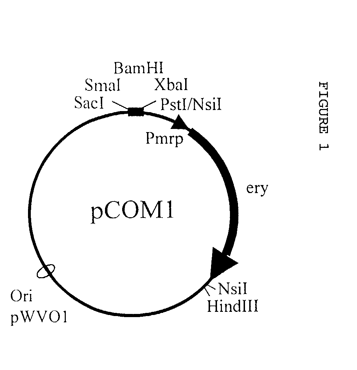

pCOM1. pCOM1 (Fig. 1) is based on the replication functions of pWVO1 (13). Moreover the vector contained the erythromycin-resistance gene of pE194 (11) preceded by the promoter region of the *mrp* gene (25) as well as the *Sac*I*-Pst*I part of the multiple cloning site of pKUN19 (14). As the result pCOM1 contained a unique *Bam*HI site (Fig. 1).

Construction of genomic *S. suis* library in pCOM1. *Sau*3AI partial digests of the DNA of the pathogenic *S. suis* serotype 2 strain 10 were size fractionated (> 3 kb) by precipitation with 4.6% of PEG 6000 (BDH Chemicals, 19). The fragments were ligated to *Bam*HI digested pCOM1 and the ligation mixtures were transformed to *E. coli* XL2-blue cells. Erythromycin-resistant colonies were selected. About 17000 independent *E*. *coli* clones were obtained. Analysis of 55 of the transformants showed that 64% contained an insert > 3 kb. From the pool of *E*. *coli* transformants plasmid DNA was isolated, which was subsequently used for the electrotransformation of the weak-pathogenic *S*. *suis* strain S735 (27). This resulted in approximately 30.000 independent *S*. *suis* transformants. The *S*. *suis* library was designated S735(pCOM-L). The transformants were pooled and stored at -80°C.

DNA techniques. Routine DNA manipulations were performed as described by Sambrook et al. (22). DNA sequences were determined on a 373A DNA Sequencing System (Applied Biosystems, Warrington, GB). Samples were prepared by use of an ABI/PRISM dye terminator cycle sequencing ready reaction kit (Applied Biosystems). Custom-made sequencing primers were purchased from Life Technologies. Sequencing data were assembled and analyzed using the McMollyTetra software package. The BLAST program was used to search for protein sequences homologous to the deduced amino acid sequences.

For PCR reaction mixtures (50 µl) the PCR Expand High Fidelity system (Boehringer, Mannheim, Germany) was used as described by the supplier. DNA amplification was carried out in a Perkin Elmer 9600 thermal cycler and the program consisted of an incubation for 2 min at 95°C, 10 cycles of 20 sec at 95°C, 1 min at 60°C and 4 min at 68°C, 30 cycles of 20 sec at 95°C, 1 min at 60°C and 4 min, extended with 20 sec for each cycle, at 68°C and 10 min at 72°C.

Southern blotting and hybridization. Chromosomal DNA was isolated as described by Sambrook et al. (22). DNA fragments were separated on 0.8% agarose gels and transferred to Gene-Screen Plus membranes (NEN) as described by Sambrook et al. (22). DNA probes were labeled with [(α-³²P]dCTP (3000 Ci mmol⁻¹; Amersham) by use of a random primed labeling kit (Boehringer). The DNA on the blots was hybridized at 65°C with the appropriate DNA probes as recommended by the supplier of the Gene Screen Plus membranes. After hybridization, the membranes were washed twice with a solution of 40 mM sodium phosphate, pH 7.2, 1 mM EDTA, 5% SDS for 30 min at 65°C and twice with a solution of 40 mM sodium phosphate, pH 7.2, 1 mM EDTA, 1% SDS for 30 min at 65°C.
Construction of pCOM-V10-ORF2 and pCOM-V10-ORF3.
To construct pCOM-V10-ORF2 we used the primers 5'-CGAGCTCGGAAGAATTGGTTATTGCGCGTG-3' and 5'-CGGGATCCCGGGGGATGACCTGTTGCTTG-3', in a PCR reaction on chromosomal DNA of *S*. *suis* strain 10 to amplify the ORF 2 encoding region. The resulting fragment was purified, digested with *Sac*I and *Bam*HI and cloned into *Sac*I and *Ba*mHI-digested pCOM1. To construct pCOM-V10-ORF3 we used the primers 5'-TCCCCCGGGGGACAAGCAACGGGTCATCCCC-3' and 5'-CGGGATCCCGGTTGAATGCCCGGCAAAGCG-3' to amplify the ORF 3 encoding region. The resulting fragment was digested with *Sma*I and *Bam*HI and cloned into pKUN19. The resulting plasmid was designated pKUN-ORF3. Because the ORF 2 and ORF 3 encoding regions are most probably co-transcibed, we.subsequently amplified the promoter region of ORF 2 by using the primers 5'-CGAGCTCGGAAGAATTGGTTATTGCGCGTG-3' and
5'-TCCCCCGGGGGAGTCGTGTGTATTCGACAGCGG-3'. The fragment was digested with *Sac*I and *Sma*I and cloned into *Sac*I and *Sma*I digested pKUN-ORF3. The resulting plasmid was digested with *Sac*I and *Bam*HI, the insert fragement was purified and cloned into *Sac*I and *Bam*HI digested pCOMI. This resulted in pCOM-V10-ORF3.

Experimental infections. Germfree pigs, crossbreeds of Great Yorkshire and Dutch landrace, were obtained from sows by caesarian sections. The surgery was performed in sterile flexible film isolators. Pigs were allotted to groups, each consisting of 4 or 5 pigs, and were housed in sterile stainless steel incubators. Housing conditions and feeding regimes were as described before (30, 33). One week old pigs were inoculated intravenously with *S*. *suis* strains as described before (30, 33). Pigs received erythromycin twice a day orally (Erythromycin stearate, Abbott B.V., Amstelveen, The Netherlands, 40 mg/kg body weight). Two hours after the infection the pigs were treated with erythromycin for the first time. Pigs were monitored twice a day for clinical signs of disease, such as fever, nervous signs and lameness. Blood samples were collected three times a week from each pig. White blood cells were counted with a cell counter. To monitor infection with *S. suis* we collected swabs of nasopharynx and feces daily. The swabs were plated directly onto Columbia agar containing 6% horse blood. After the pigs were killed, they were examined for pathological changes. Moreover, tissue specimens were collected from the central nervous system, serosae, joints, lungs, liver, kidney, spleen, heart and tonsils. The tissues were homogenized in the presence of Todd-Hewitt medium by using an Ultra-Turrax tissuemizer (Omni International, Waterbury, USA), centrifuged for 5 min at 3000 rpm and the supernatants were frozen at -80° C in the presence of 15% glycerol.

### RESULTS

Complementation system. A genomic library of the pathogenic S. *suis* strain 10 was constructed into the weak-pathogenic strain S735 as described in Materials and Methods. The plasmid pCOM1 allowed the insertion of large DNA fragments into the unique *Bam*HI site (Fig. 1). The plasmid carries the origin of replication of pWVO1, which functions in *E. coli* as well as in *S*. *suis* (27). This allowed the construction of a DNA library in *E. coli* first. Plasmid DNA, isolated from the pool of *E. coli* transformants, was subsequently electrotransformed into S. suis strain S735. 30.000 individual *S*. *suis* clones were obtained. As determined by analysis of 24 randomly selected transformants, more than 30% of the S735(pCOM-L) transformants contained an insert > 3kb.

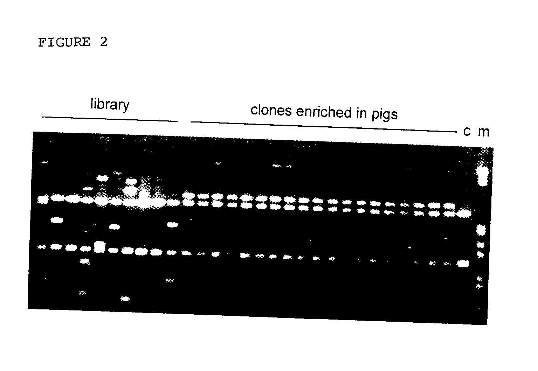

Selection of genomic fragments associated with virulence. To select for genetic determinants of the pathogenic *S. suis* strain 10 that could increase the virulence of the weak-pathogenic strain S735, pigs were inoculated with the S. *suis* library S735(pCOM-L). We used a dose of either 10⁷ or 10⁸ cfu, and the pigs were treated with erythromycin as described in Materials and Methods. All pigs showed specific *S. suis* symptoms (Table 2A) three to seven days after the infection and except for one, all pigs died during the course of the experiment. From five of the pigs bacteria could be reisolated from the CNS and from two other pigs bacteria were isolated from the joints (Table 2A). In previous experiments, in which pigs were inoculated with weak-pathogenic strains, specific *S. suis* symptoms were observed with a very low frequency (33, 34). In addition, from those pigs bacteria could never be re-isolated from the CNS nor from the joints. Therefore, these data indicated that compared to virulence of strain S735, bacteria isolated from pigs inoculated with the *S. suis* library S735(pCOM-L) are more virulent due to the presence of a DNA fragment of the pathogenic strain 1Q. The plasmid content of 90 randomly selected clones isolated from the CNS or the joints of the seven diseased pigs was analyzed by PCR and restriction analysis. The results showed that 88 of the 90 clones analyzed (19 of which are shown in Fig. 2) contained an insert of about 3 kb and had identical restriction patterns. Moreover, the inserts of 10 randomly selected clones, having identical restriction patterns, also showed identical DNA sequences (results not shown). Plasmid DNA of 10 randomly selected clones from the original S735(pCOM-L) library showed 10 different restriction patterns (Fig. 2). These data suggest that one specific clone, which was designated S735(pCOM-V10), was greatly enriched in seven different pigs. Moreover, this particular clone was isolated from the CNS as well as from the joints of the various pigs, indicating that the observed enrichment was not tissue specific.

Virulence-associated properties of the selected fragment V10. To further analyze the virulence properties of strain S735(pCOM-V10), pigs were inoculated intravenously with 10⁶ cfu of strain S735(pCOM1) or strain S735(pCOM-V10). The results (Table 2B) clearly show that, compared to the virulence of strain S735(pCOM1), the virulence of strain S735(pCOM-V10) was greatly enhanced. All pigs inoculated with strain S735(pCOM-V10) showed specific *S*. *suis* symptoms and died within one day after infection. In contrast, except for one, none of the pigs inoculated with the control strain S735(pCOM1) showed specific clinical symptoms and these pigs survived until the end of the experiment (15 days after infection). These data proved that introduction of fragment V10 of strain 10 into S735 transformed the weak-pathogenic strain S735 into a highly pathogenic strain. This strongly suggests that the protein(s) encoded by V10 are important virulence determinants and play an important role in the pathogenesis of *S. suis* serotype 2 infections in pigs.

To find out whether the observed increase of the fragment V10 on virulence was specific for strain S735, we introduced pCOM1 and pCOM-V10 into another weak-pathogenic strain: strain 24 (33). Subsequently we determined the virulence properties of the strains 24(pCOM1) and 24(pCOM-V10). As shown in Table 2C and 2D, similar effects of V10 on the virulence of strains S735 and 24 were observed. Both strains 24(pCOM-V10) and S735(pCOM-V10) were highly pathogenic for young piglets, whereas strains 24(pCOM1) and S735(pCOM1) were shown to be only weak-pathogenic (Table 2C and D). This strongly indicates that V10 has a more general ability to transform weak-pathogenic serotype 2 strains into highly pathogenic strains.

Because we used a plasmid system for the complementation approach, gene-doses effects cannot be excluded. Plasmid pCOM1 is based on the replication functions of pWVO1. In Gram-positive bacteria the latter plasmid has a copy number between 3 and 6 (13). To find out whether copy effects play a role, we cloned the genomic region of strain S735 homologous to fragment V10 of strain 10 (see below) into plasmid pCOM1. This plasmid was designated pCOM-V735. The virulence of strains S735(pCOM-V735), and 24(pCOM-V735) was subsequently compared to that of S735(pCOM-V10), S735(pCOM1), 24(pCOM-V10) and 24(pCOM1). The results (Table 2C and D) clearly show that, in contrast to pCOM-V10, the plasmid pCOM-V735, did not carry virulence-enhancing activity. Pigs infected with strains S735(pCOM-V10) and 24(pCOM-V10) died within one or two days after infection, whereas most of the pigs infected with strains S735(pCOM-V735), 24(pCOM-V735), S735(pCOM1) and 24(pCOM1) survived until the end of the experiment (17 days after infection). Compared to pigs infected with strains containing pCOM1, pigs infected with strains containing pCOM-V735 developed more general and specific signs of disease, but much less than pigs infected with strains containing pCOM-V10 (Table 2C and D). From these data we concluded that the differences in virulence observed between the strains containing pCOM-V10 and the strains containing pCOM-VS735 are caused by differences between the fragments V10 and V735 (see below). The differences in virulence observed between the strains containing pCOM1 and the strains containing pCOM-VS735 may be due to gene-doses effects.

Sequence analysis of fragments V10 and V735. By using the fragment V10 as a probe a 3.1-kb *Pst*I*-Hin*dIII fragment of strain S735 (V735) was identified and cloned into pCOM1 (Fig. 3). To analyze the differences between the fragments V10 and V735, we determined the nucleotide sequences of the fragments V10 and V735 and analyzed the sequences for homology to known genes by comparison with the GenBank/EMBL and SWISSPROT databases. The sequence of V10 revealed two complete and two incomplete open reading frames (Fig. 3). ORF 1 (nucleotides 1 to 461) coded for a polypeptide of 153 amino acids. This protein showed homology (49% identity) to the C-terminal region of acetate kinase of *Clostridium thermocellum* (accession number AF041841) and various other bacterial species (12). ORF 2 (nucleotides 625 to 1327) coded for a protein of 233 amino acids. No significant similarities were found between the predicted amino acid sequence of this protein and proteins present in the datalibraries. ORF 3 (nucleotides 1382 to 2639) coded for a protein of 418 amino acids. This protein showed homology (36% identity) to FolC (folylpolyglutamate synthetase) of *Bacillus subtilis* (15, 17). Compared to the other ORFs, ORF 4 is transcribed in the opposite direction. ORF 4 (nucleotides 2684 to 2972) coded for a polypeptide of 96 amino acids. This polypeptide showed homology (67% identity) to the C-terminal part of PepA (glutamyl-aminopeptidase) of *Lactococcus lactis* (1). Both ORFs 2 and 3 possessed putative initiation codons and ribosome-binding sites. Putative -35 (TGGACA) and -10 (TACAAT) sequences, which may function as promoter sequences, were found preceding ORF 2. ORFs 2 and 3 were separated by 55 nucleotides. In this region no putative promoter sequences could be observed. This could indicate that the ORFs 2 and 3 are co-transcribed. Downstream of the ORFs 1 and 3 we found regions of extended dyad symmetry, which may probably function as transcription termination signals.

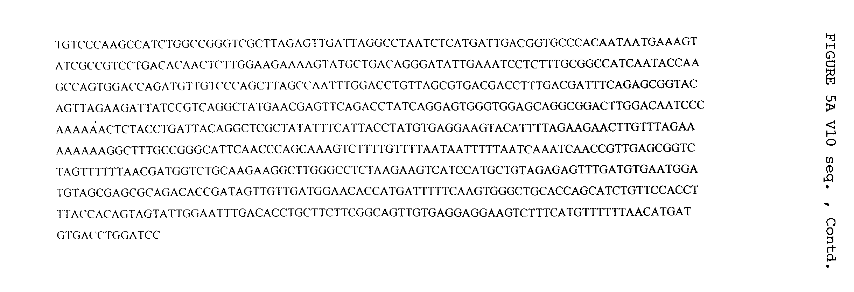

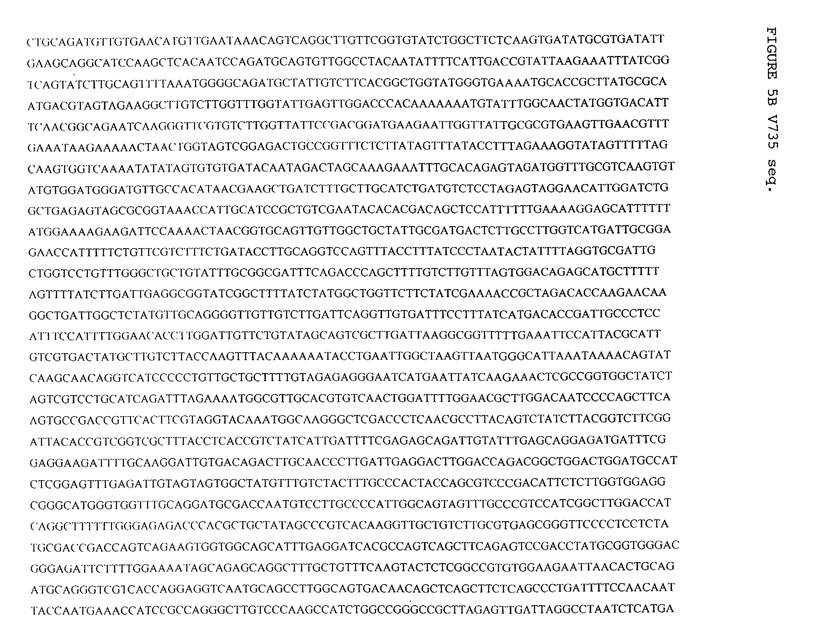

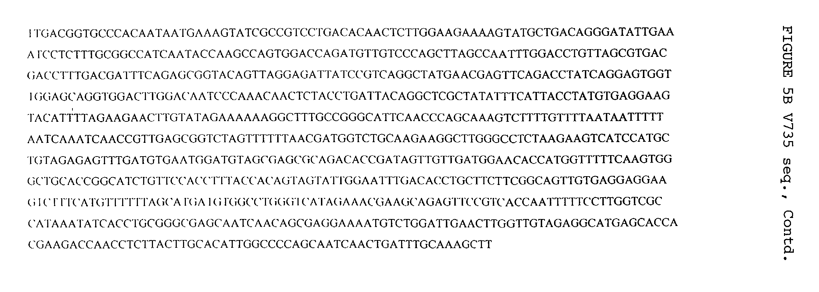

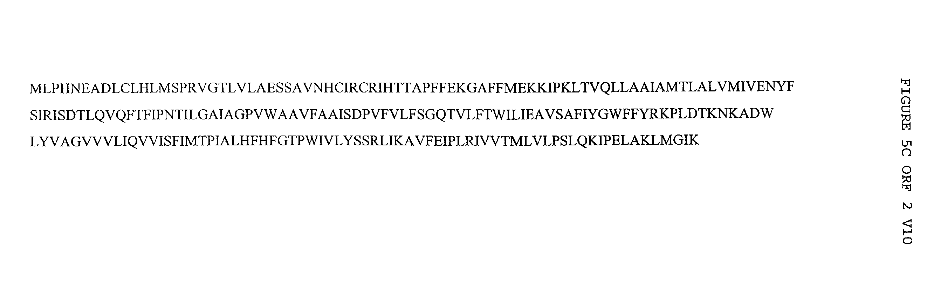

The sequence of the fragment V735 was determined and compared to the sequence of the fragment V10. No major deletions or insertions were found between the sequenced regions. The ORFs 1, 3 and 4 of strains 10 and S735 were highly homologous. The putative protein fragments encoded by the ORFs 1 differed in 2 (1,3%) amino acids; the putative proteins encoded by the ORFs 3 differed in 19 (4.5%) amino acids (Fig. 4B), whereas the putative protein fragments of the ORFs 4 were identical. However, major differences were observed between the ORFs 2 of strains 10 and S735. In the pathogenic strain 10 an ORF of 699 bases was found, predicting a protein product of 233 amino acids. In contrast, due to a frame-shift mutation, in the weak-pathogenic strain S735 an ORF of 569 bases was found, coding for a polypeptide of 183 amino acids. Compared to the putative protein encoded by strain 10, the putative protein encoded by strain S735 lacked the N-terminal 50 amino acids (Fig. 4A). Beside these N-terminal differences, the putative proteins differed at 9 amino acid positions (4.9%). In addition, the putative -35 regions that may be part of the promoter sequences, involved in the expression of ORFs 2 and 3, differed between the two strains. A TGGACA sequence was found in strain 10, whereas a TGGTCA sequence was found in strain S735. The sequence data suggest that the differences in the virulence-enhancing effects of the fragments V10 and V735 may be the results of functional differences between the putative proteins expressed by the ORFs 2 and/or 3, and/or by differences in their levels of expression. ORF 2 or ORF 3?

To examine whether the observed increase of the fragment V10 on virulence resulted from ORF 2 or ORF 3 or both, we next constructed the plasmids pCOM-V10-ORF2 and pCOM-V10-ORF3, containing the individual ORF 2 and ORF 3 encoding regions. Because ORF 3 most probably is co-transcibed with ORF 2, in pCOM-V10-ORF3 the ORF 3 encoding region was preceded by the promoter region of ORF 2. Subsequently we determined the virulence properties of the strains S735(pCOM-V10), S735(pCOM-V10-ORF2), S735(pCOM-V10-ORF3) and S735(pCOM1). As shown in Table 2E the fragements V10 and ORF 2 showed similar effects on the virulence of strain S735. No effect of ORF 3 on the virulence of strain S735 could be observed. These data show that ORF 2 is responsible for the observed effect on virulence and that the ORF 2 protein is an important virulence factor.

Distribution of the *orf2* and *orf3* sequences among all known 35 *S. suis* serotypes. To examine the homology between the *orf2* and *orf3* genes and genes of other *S*. *suis* serotypes, we performed cross-hybridization experiments. DNA fragments of the *orf2* and 3 genes were amplified by PCR, labelled by ³²P, and hybridized to chromosomal DNAs of the reference strains of the 35 different *S. suis* serotypes. As a positive control we used a probe specific for 16S rRNA (24). The 16S rRNA probe hybridized with almost equal intensities with all serotypes tested (results not shown). Probes *orf2* and *orf3* hybridized with all serotypes, except for serotypes 32 and 34 (results not shown). This indicates that the proteins encoded by *orf2* and 3 are common among most *Streptococcus species*

Herein we thus provide the development and the successful application of an *in vivo* complementation approach for the identification of important molecular determinants that determine the differences in virulence between pathogenic and weak-pathogenic strains of *Streptococcus.* Using the complementation approach one unique clone, containing a 3.0-kb fragment of pathogenic strain (V10), was selected. The selected fragment was greatly enriched in seven different pigs and the observed enrichment was not tissue specific. The selected fragment showed similar enhancing effects on the virulence of two different weak-pathogenic strains. Large differences were observed between the effects of the selected fragment V10 of the pathogenic strain 10 and the corresponding fragment V735 isolated from the weak-pathogenic strain S735 on virulence. In contrast to V10, which had a strong virulence-enhancing effect on weak-pathogenic strains, V735 showed only minor effects. Therefore, differences between these two fragments are considered responsible for the observed differences on virulence. Sequence data showed that the fragments V10 and V735 were highly homologous. Both fragments contained two complete ORFs (ORFs 2 and 3), both of which can potentially express proteins that may further contribute to the observed effect on virulence. The ORFs 3 are highly homologous and differ in only 19 amino acids. The proteins encoded by the ORFs 3 showed homology to FolC (folylpolyglutamate synthetase) of various pro- and eucaryotic organisms. Folylpolyglutamate synthetase catalyzes the conversion of folates to polyglutamate derivatives (4). Bacteria require folates for the biosynthesis of glycin, methionine, formylmethionine, thymidine, purines and patothenate (4). Whether the FolC proteins encoded by the fragments V10 and V735 have different enzymatic activities or different substrate specificities is unknown so far. In *E. coli a folC* mutant is methionine deficient (4), however, so far a role of FolC in virulence has not been described. Significant differences were also observed between the ORFs 2 of the fragments V10 and V735. Compared to the putative ORF 2 protein encoded by strain 10, the putative protein encoded by strain S735 lacked the N-terminal 50 amino acids. In strain S735 a strong ribosome-binding site precedes the methionine startcodon of ORF 2. In contrast, however, in strain 10 the sequence did not indicate the presence of a strong ribosome-binding site preceding the methionine startcodon of ORF 2. Therefore, although ORF 2 of strain 10 is extended compared to ORF 2 of strain S735, it is not clear whether the proteins expressed by these two ORFs differ in length. Future experiments will be required to analyze the expressed proteins in detail. In addition to the putative N-terminal differences, the putative ORF 2 proteins differed at 9 amino acid positions (4.9%). Except for one, these amino acid substitutions were clustered at two different positions in the putative protein. The function of the ORF 2 protein is unknown so far. Not even distant or partial homologies were found between the ORF 2 protein sequences and protein sequences present in the data libraries. Hydrophobicity profiles showed that the ORF 2 encoded protein(s) is very hydrophobic. A role of the ORF 2 protein in the cellular membrane is therefore suggested. The putative -35 region preceding the ORFs 2 and 3 differed between strains S735 and 10. Therefore, we cannot exclude that differences in the expression levels rather than functional differences are responsible for the observed effects on virulence.

In previous experiments we found that pigs infected with weak-pathogenic strains showed only mild clinical signs of disease and that bacteria could never be reisolated from the CNS or the joints (33, 34). Surprisingly, in the experiments described in this paper, in which we used weak-pathogenic strains containing the control plasmid pCOM1, bacteria could (with a low frequency) be re-isolated from the CNS as well as from the joints. There are several possible explanations for these observed differences. One explanation is that the presence of the plasmid somehow affects the (virulence) properties of the strains. Another possibility is that the treatment of the pigs with erythromycin makes the pigs more sensitive for *S. suis* infections and a third possibility is that compared to the pigs used previously, the pigs used for the current experiments were more sensitive for *S. suis* infections. References
1. lAnson, K. J., S. Movahedi, H. G. Griffin, M. J. Gasson, and F. Mulholland. 1995. A non-essential glutamyl aminopeptidase is required for optimal growth of *Lactococcus lactis* MG1363 in milk. Microbiol. 141: 2873-2881.
2. Arends, J. P., and H. C. Zanen. 1988. Meningitis caused by *Streptococcus suis* in humans. Rev. Infect. Dis. 10: 131-13.
3. Awad-Masalmeh, M., J. Köfer, M. Schuh, and F. Hinterdorfer. 1999. Serotypen, virulenzfaktoren und empfindlichkeit gegenuber antibiotika von *Streptococcus suis* stämmer isoliert aus klinisch gesunden und erkrankten schweinen in Österreich. Wien. Tierärtztl. Mschr. 86: 262-269.
4. Bogner, A. L., C. Osborne, and B. Shane. 1987. Primary structure of the *Escherichia coli folC* gene and its folylpolyglutamate synthetase-dihydrofolate synthetase product and regulation of expression by an upstream gene. J. Biol. Chem. 262: 12337-12343.
5. Chalettier, S., M. Gottschalk, R. Higgins, R. Brousseau and J. Harel. 1999. Relatedness of *Streptococcus suis* serotype 2 isolates from different geographic origins as evaluated by molecular fingerprinting and phenotyping. J. Clin. Microbiol. 37: 362-366.
6. Clifton-Hadley, F.A. 1983. *Streptococcus suis* type 2 infections. Br. Vet. J. 139: 1-5.
7. Galina, L., U. Vecht, H. J. Wisselink, and C. Pijoan. 1996. Prevalence of various phenotypes of *Streptococcus suis* isolated from swine in the U.S.A. based on the presence of muramidase-released protein and extracellular factor. Can. J. Vet. Res. 60: 72-74.8.
8. Gottschalk, M., R. Higgins, M. Jacques, R. K. Mittal, and J. Henrichsen. 1989 Description of 14 new capsular types of *Streptococcus suis* J. Clin. Microbiol. 27: 2633-2636.
9. Gottschalk, M., R. Higgins, M. Jacques, M. Beaudain, and J. Henrichsen. 1991. Characterization of six new capsular types (23-28) of *Streptococcus suis.* J. Clin. Microbiol. 29: 2590-2594.
10. Higgins, R., M. Gottschalk, M. Jacques, M. Beaudain, and J. Henrichsen. 1995. Description of six new capsular types (29-34) of *Streptococcus* suis. J. Vet. Diagn. Invest. 7: 405-406.
11. Horinouchi, S., and B. Weisblum. Nucleotide sequence and functional map of pE194, a plasmid that specifies inducible resistance to macrolide, lincosamide and streptogramin type B antibiotics. J. Bacteriol. 150: 804-814.
12. Kakuda, H., K. Honoso, K. Shiroishi, and S. Ichihara. 1994. Identification and characterization of the ack (acetate kinase A)-pta (phosphotransacetylase) operon and complementation analysis of acetate utilization by an ackA-pta deletion mutant of *Escherichia coli.* J. Biochem. 116: 916-922.
13. Kok, J., J. M. B. M. van der Vossen, and G. Venema. 1984. Construction of plasmid cloning vectors for lactic acid streptococci which also replicate in *Bacillus subtilis* and *Escherichia coli.* Appl. Environ. Microbiol. 48: 726-731.
14. Konings, R. N. H., E. J. M. Verhoeven, and B. P H. Peeters. 1987. pKUN vectors for the separate production of both DNA strands of recombinant plasmids. Methods Enzymol. 153: 12-34.
15. Luo, D., J. Leautey, M. Grunberg-Manago, and H. Putzer. 1997. Structure and regulation of expression of the *Bacillus subtilis* valyl-tRNA synthetase gene. J. Bacteriol. 179: 2472-2478.
16. Luque, I., C. Tarradas, R. Astorga, A. Perea, H. J. Wisselink, and U. Vecht. 1998. The presence of muramidase released protein and extracellular factor protein in various serotype of *Streptococcus suis* isolated from diseased and healthy pigs in Spain. Res. Vet. Science 66: 69-72.
17. Margolis, P. S., A. Driks, and R, Losick. 1993. Sporulation gene spoIIB from *Bacillus subtilis.* J. Bacteriol. 175: 528-540.
18. Miller, J. 1972. Experiments in molecular genetics. Cold Spring Harbor Laboratory, Cold Spring Harbor, N. Y.
19. Paithankar, K. R., and K. S. N. Prasad. 1991. Precipitation of DNA by polyethylene glycol and ethanol. Nucleic Acids Res. 19: 134.
20. Sambrook, J., E. F. Fritsch, and T. Maniatis. 1989. Molecular cloning: a laboratory manual, 2nd ed. Cold Spring Harbor Laboratory Press, Cold Spring Harbor, N. Y.
21. Salasia, S.I.O., and C. Lämmler. 1995. Distribution of serotype, virulence markers and further characteristics of *Streptococcus suis* isolates from pigs. J. Vet. Med. Series B 42: 78-83.
22. Sambrook, J., E. F. Fritsch, and T. Maniatis. 1989. Molecular cloning: a laboratory manual, 2nd.ed. Cold Spring Harbor Laboratory Press, Cold Spring Harbor, N. Y.
23. Smith, H. E., M. Damman, J van der Velde, F. Wagenaar, H. J. Wisselink, N. Stockhofe-Zurwieden, and M. A. Smits. 1999. Identification and characterization of the *cps* locus of *Streptococcus suis* serotype 2: the capsule protects against phagocytosis and is an important virulence factor. Infect. Immun. 67: 1750-1756.
24. Smith, H. E., M. Rijnsburger, N. Stockhofe-Zurwieden, H. J. Wisselink, U. Vecht, and M. A. Smits. 1997. Virulent strains of *Streptococcus suis* serotype 2 and highly virulent strains of *Streptococcus suis* serotype 1 can be recognized by a unique ribotype profile. J. Clin. Microbiol. 35: 1049-1053.
25. Smith, H. E., U. Vecht, A. L. J. Gielkens, and M. A. Smits. 1992. Cloning and nucleotide sequence of the gene encoding the 136-kilodalton surface protein (muramidase-released protein) of *Streptococcus suis* type 2. Infect. Immun. 60: 2361-2367.
26. Smith, H. E., U. Vecht, H. J. Wisselink, N. Stockhofe-Zurwieden, Y. Biermann, and M. A. Smits. 1996. Mutants of *Streptococcus suis* types 1 and 2 impaired in expression of muramidase-released protein and extracellular protein induce disease in newborn germfree pigs. Infect Immun. 64: 4409-4412.
27. Smith, H. E., H. J. Wisselink, U. Vecht, A. L. J. Gielkens, and M. A. Smits. 1995. High-efficiency transformation and gene inactivation in *Streptococcus suis* type 2. Microbiol. 141: 181-188.
28. Staats, J. J., B. L. Plattner, G. C. Stewart, and M. M. Chengappa. 1999. Presence of the *Streptococcus suis* suilysin gene and expression of MRP and EF correlates with high virulence in *Streptococcus suis* type 2 isolates. 1999. Vet. Microbiol. 70: 201-211.
29. Stockhofe-Zurwieden, N., U. Vecht, H. J. Wisselink, H. van Lieshout, and H. E. Smith. 1996. Comparative studies on the pathogenicity of different *Streptococcus suis* serotype 1 strains, p299. *In* P. G. Monetti and G. Vignola (ed.), Proceedings of the 14th International Pig Veterinary Society Congress, Bologna, Italy.
30. Vecht, U., J. P. Arends, E. J. van der Molen, and L. A. M. G. van Leengoed. 1989. Difference in virulence between two strains of *Streptococcus suis* type 2 after experimentally induced infection of newborn germfree pigs. Am. J. Vet. Res. 50: 1037-1043.
31. Vecht, U., L. A. M. G. van Leengoed, and E. R. M. Verheyen. 1985. *Streptococcus suis* infections in pigs in The Netherlands (part one). Vet. Q. 7: 315-321.
32. Vecht, U., H. J. Wisselink, M. L. Jellema, and H. E. Smith. 1991. Identification of two proteins associated with virulence of *Streptococcus suis* type 2. Infect. Immun. 59: 3156-3162.
33. Vecht, U., H. J. Wisselink, J. E. van Dijk, and H. E. Smith. 1992. Virulence of *Streptococcus suis* type 2 strains in newborn germfree pigs depends on phenotype. Infect. Immun.60: 550-556.
34. Vecht, U., H. J. Wisselink, N. Stockhofe-Zurwieden, and H. E. Smith. 1995. Characterization of virulence of the *Streptococcus suis* serotype 2 reference strain Henrichsen S735 in newborn germfree pigs. Vet. Microbiol. 51: 125-136.
35. Wisselink, H. J., H. E. Smith, N. Stockhofe-Zurwieden, K. Peperkamp and U. Vecht. 2000. Distribution of capsular types and production of muramidase-released protein (MRP) and extracellular factor (EF) of *Streptococcus suis* strains isolated from diseased pigs in seven European Countries. Vet. Microbiol.74: 237-247.

**Table 1.**

| Bacterial strains and plasmids | | |
|---|---|---|
| strain/plasmid | relevant characteristics* | source/reference |
| Strain | | |
| *E. coli* | | |
| XL2 blue | | Stratagene |
| | | |
| *S. suis* | | |
| 10 | pathogenic serotype 2 strain | (33) |
| S735 | weak-pathogenic serotype 2 reference strain | (34) |
| 24 | weak-pathogenic serotype 2 strain | (33) |
| | | |
| Plasmid | | |
| pKUN19 | replication functions pUC, Amp^{R} | (14) |
| pE194 | Em^{R} | (11) |
| pMR11 | pKUN19 containing *S*. *suis mrp* gene | (25) |
| pCOM1 | replication functions pWVO1, Em^{R} | this work |
| pCOM-L | pCOM1 containing random sequences of *S. suis* strain 10 | this work |
| pCOM-V10 | pCOM1 containing *S*. *suis* strain 10 fragment selected in pigs | this work |
| pCOM-V735 | pCOM1 containing a 3.1 kb *Pst*I*-Hin*dIII fragment from *S*. *suis* strain S735 (homologous to V10) | this work |

| | | |
|---|---|---|
| * Spc^{R}: spectinomycin resistant Amp^{R}: ampicillin resistant Em^{R}: erythromycin resistant | | |

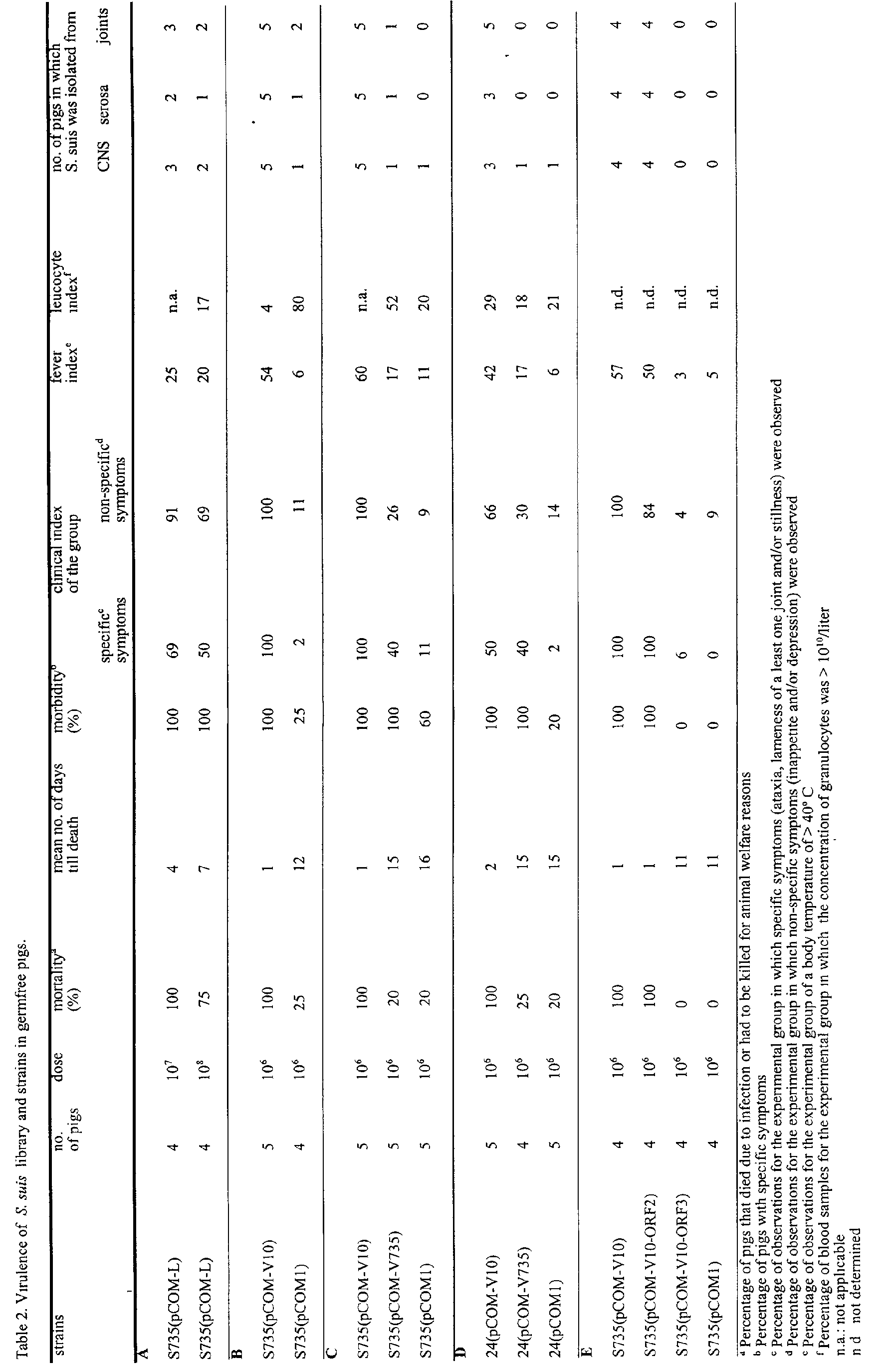

### LEGEND TO FIGURES

Fig. 1.
   The pCOM1 vector used in this study. pCOM1 contains the replication functions of pWVO1 (13), the erythromycin-resistance gene of pE194 (11) preceded by the promoter region of the *mrp* gene (25) as well as the *Sac*I*-Pst*I part of the multiple cloning site of pKUN19 (14).
Fig. 2.
   Plasmids digested with *Sma*I and *Xba*I on an 0.8% agarose gel. Library: plasmids isolated from 10 randomly selected clones of the original library; clones enriched in pigs: plasmids isolated from 19 independently selected clones enriched in pigs; c: pCOM1; m: molecular size marker.
Fig. 3.
   Schematic representation of the fragments V10 and V735. The arrows indicate the potential ORFs. 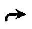 P, indicates the position of the potential promoter sequence; °, indicate the positions of the potential transcription regulator sequences. Homologies (% identities) between the potential proteins encoded by the ORFs and proteins present in the data libraries are indicated.
Fig. 4. A, B
   Homology between the ORFs2 (A) and 3 (B) encoding proteins of fragments V10 and V735. The asterisks indicate the non-identical amino acids.
Fig. 5. A, B, C, D, E, F
   Nucleotide and amino acid sequences of fragments of V10 and V735.

## Claims

1. A method for modulating virulence of a *Streptococcus* comprising modifying a genomic fragment of said *Streptococcus* wherein said genomic fragment comprises at least a functional part of a fragment identifiable in *Streptococcus suis* as a nucleic acid or fragment thereof as shown in figure 5.

2. A method for assaying virulence of a *Streptococcus* comprising assaying a genomic fragment of said *Streptococcus* wherein said genomic fragment comprises at least a functional part of a fragment identifiable in *Streptococcus suis* as a nucleic acid or fragment thereof as shown in figure 5.

3. An isolated and/or recombinant nucleic acid derivable from *Streptococcus* and identifiable in *Streptococcus suis* as a nucleic acid or fragment thereof as shown in figure 5.

4. A vector comprising a nucleic acid according to claim 3

5. A host cell comprising a nucleic acid according to claim 3 or a vector according to claim 4.

6. A host cell according to claim 5 comprising a *Streptococcus.*

7. A vaccine comprising a nucleic acid according to claim 3 or a vector according to claim 4 or a host cell according to claim 5 or 6.

8. A protein or fragment thereof encoded by a nucleic acid according to claim 3.

9. An antibody directed against a protein or fragment thereof according to claim 8.

10. An antigen comprising a protein or fragment thereof according to claim 8

11. A diagnostic test comprising an antibody according to claim 9.

12. A vaccine or diagnostic test comprising an antigen according to claim 10.
